# EUROPÄISCHE PATENTANMELDUNG

(11) **EP 2 695 656 A1**
(43) Veröffentlichungstag der Anmeldung: **12.02.2014**
(21) Anmeldenummer: 12179912.6
(22) Anmeldetag: 09.08.2012
(51) Int. Cl.: B01D 33/01, G01N 33/49, B01L 3/00

(54) **Verfahren sowie eine Separationsvorrichtung zur Abtrennung eines Filtrats von einer Probenflüssigkeit**

(71) Anmelder: F. Hoffmann-La Roche AG, 4070 Basel (CH); Roche Diagnostics GmbH, 68305 Mannheim (DE)
(72) Erfinder: Huemer, Herfried, 8330 Feldbach (AT)
(74) Vertreter: Babeluk, Michael

(57) **Zusammenfassung**

Die Erfindung betrifft eine Separationsvorrichtung zur Abtrennung eines Filtrats von einer Probenflüssigkeit, insbesondere zur Gewinnung von Plasma (42) aus Vollblut (41), mit einem Probenbehälter (25) zur Aufnahme der Probenflüssigkeit und einem in den Probenbehälter (25) dichtend einführbaren Filterkolben (26), der stirnseitig ein Filterelement (24) und an der gegenüberliegenden Seite ein Griffelement (27) aufweist und in seinem Inneren das gewonnene Filtrat aufnimmt. Erfindungsgemäß ist nach dem Einsetzen des Filterkolbens (26) in den Probenbehälter (25) zwischen der Innenwand des Probenbehälters (25) und der Außenwand des Filterkolbens (26) eine nach außen durch eine Dichtlippe (32) abgeschlossene Ringkammer (34) ausgebildet, in der sich beim Einführen des Filterkolbens (26) in den Probenbehälter (25) ein auf die Probenflüssigkeit wirkendes Luftpolster ausbildet, wobei eine Strömungsverbindung von der Ringkammer (34) zur Stirnseite des Filterelements (24) vorgesehen ist.

## Beschreibung

Die Erfindung betrifft ein Verfahren sowie eine Separationsvorrichtung zur Abtrennung eines Filtrats von einer Probenflüssigkeit, insbesondere zur Gewinnung von Plasma aus Vollblut, mit einem Probenbehälter zur Aufnahme der Probenflüssigkeit und einem in den Probenbehälter dichtend einführbaren Filterkolben, der stirnseitig ein Filterelement und an der gegenüberliegenden Seite ein Griffelement aufweist und in seinem Inneren das gewonnene Filtrat aufnimmt.

Neben den vor allem in Labors verwendeten Zentrifugen zur Plasmaabtrennung sind bereits eine Reihe von Vorrichtungen bekannt geworden, bei welchen auch im POC (Point of Care) -Bereich kleinste Plasmamengen durch Abtrennung aus Vollblut mittels Filtern zur Verfügung gestellt werden.

Im einfachsten Fall kann die Plasmaabtrennung durch einen mehrschichtigen Teststreifen gemäß DE 40 15 589 A1 (BOEHRINGER MANNHEIM) erfolgen, welcher auf einer inerten Trägerfolie eine Transportschicht zum Transport der Probenflüssigkeit (Vollblut) von einem Aufgabebereich in einen Messbereich aufweist. Die Transportschicht kann beispielsweise aus einem Glasfaservlies bestehen, das im Aufgabebereich von einer Plasmaabtrennschicht bedeckt ist. Das Verfahren eignet sich allerdings nur für Analysatoren, die mit Teststreifen arbeiten.

Von WHATMAN INC. Florham Park, NJ 07932 USA ist eine Separationsvorrichtung mit der Bezeichnung "Mini-UniPrep" bekannt, die geeignet ist, Proben für die High Performance Liquid Chromatography (HPLC) vorzubereiten. Die ungefilterte Probe wird in einen Probenbehälter gefüllt und danach ein Filtrierkolben eingesetzt, der stirnseitig ein Filter aufweist. Der Filtrierkolben wird in den Probenbehälter gedrückt, bis sich in seinem Inneren das abgetrennte Filtrat sammelt, wobei die verdrängte Luft durch eine Lüftungsöffnung entweicht. Die Separationsvorrichtung kann danach direkt in einen Probenwechsler eines Analysators eingesetzt werden. Die Entnahme des Filtrats kann durch ein Septum in der Verschlusskappe des Filtrierkolbens erfolgen. Nachteilig bei der bekannten Separationsvorrichtung ist die Tatsache, dass der auf das Filter im Filtrierkolben wirkende Druck nicht gleichmäßig und reproduzierbar angewandt werden kann, wobei auch für die Probe schädliche Druckspitzen nicht vermeidbar sind. Dies ist insbesondere bei der Anwendung solcher Vorrichtungen zur Plasmaabtrennung aus Vollblut nachteilig, da durch solche Druckspitzen eine Hämolyse (Zerplatzen) der roten Blutkörperchen (RBKs) hervorgerufen werden kann, welche eine unerwünschte Verunreinigung der Plasmafraktion durch die so freigesetzten Inhaltsstoffe der RBKs bewirkt.

Aufgabe der Erfindung ist es, ausgehend von einer Trennvorrichtung der oben beschriebenen Art (z.B. "Mini-UniPrep" von WHATMAN INC.) Verbesserungen vorzuschlagen, die bei einer für den Anwender unkomplizierten Handhabung eine reproduzierbare Gewinnung von Plasma proben auch aus relativ kleinen Probenmengen ermöglichen.

Diese Aufgabe wird erfindungsgemäß dadurch gelöst, dass nach dem Einsetzen des Filterkolbens in den Probenbehälter zwischen der Innenwand des Probenbehälters und der Außenwand des Filterkolbens eine nach außen durch eine Dichtlippe abgeschlossene Ringkammer ausgebildet ist, in der sich beim Einführen des Filterkolbens in den Probenbehälter ein auf die Probenflüssigkeit wirkendes Luftpolster ausbildet, wobei eine Strömungsverbindung von der Ringkammer zur Stirnseite des Filterelements vorgesehen ist. Im Gegensatz zum Stand der Technik erfolgt die Druckausübung auf die Probenflüssigkeit, beispielsweise eine Vollblutprobe, somit nicht unkontrolliert direkt, sondern langsam und gleichmäßig abfallend über das komprimierte Luftpolster, wobei die Druckverhältnisse durch die geometrischen Abmessungen (beispielsweise die Volumenverhältnisse) der einzelnen Bauteile der Separationsvorrichtung und die Kenndaten des Filterelements eingestellt und aufeinander abgestimmt werden können.

Hierbei ragt der probenseitige Rand des Filterkolbens über die Stirnseite (d.h. die mit der Probenflüssigkeit in Kontakt kommende Seite) des Filterelements hinaus und bildet eine frontseitige Benetzungskammer. Bevorzugt sind in dieser Randausnehmungen oder Zuflussöffnungen angeordnet, die eine Strömungsverbindung von der Ringkammer in die frontseitige Benetzungskammer herstellen. Die Zuflussöffnungen am unteren Rand des Filterkolbens erlauben ein Nachfließen der Blutprobe bei bereits bis zum Boden eingedrücktem Filterkolben.

Alternativ oder zusätzlich können im Boden des Probenbehälters Kerben oder nutförmige Vertiefungen angeordnet sein, die eine Strömungsverbindung von der Ringkammer zur Stirnseite des Filterelements herstellen.

Das erfindungsgemäße Verfahren zum Abtrennung eines Filtrats von einer Probenflüssigkeit, insbesondere zur Gewinnung von Plasma aus Vollblut, ist durch folgende Schritte gekennzeichnet:
- Bereitstellen eines mit Probenflüssigkeit, insbesondere Vollblut, gefüllten Probenbehälters;
- Einsetzen eines erfindungsgemäßen Filterkolbens in den Probenbehälter, sodass in einer nach außen durch eine Dichtlippe verschlossenen Ringkammer zwischen Probenbehälter und Filterkolben ein unter Überdruck stehendes Luftpolster zwischen der Dichtlippe und der Oberfläche der Probenflüssigkeit entsteht, das auf die Probenflüssigkeit wirkt;
- Durchpressen der Probenflüssigkeit durch ein im Filterkolben angeordnetes Filterelement mit Hilfe des Überdrucks in der Ringkammer, sodass ausgangsseitig des Filterelements Plasma austritt; und
- Sammeln des Plasmas in einem im Filterkolben angeordneten Filtratsammelbehälter.

Die Erfindung wird im Folgenden anhand von Zeichnungen näher erläutert. Es zeigen:
- Fig. 1: eine erfindungsgemäße Separationsvorrichtung zur Abtrennung eines Filtrats von einer Probenflüssigkeit in einer Schnittdarstellung;
- Fig. 2: eine alternative Ausführungsvariante einer erfindungsgemäßen Separationsvorrichtung; sowie die
- Fig. 3: bis Fig. 7 die Verwendung der erfindungsgemäßen Separationsvorrichtung gemäß Fig. 2 in aufeinander folgenden Verfahrensschritten.

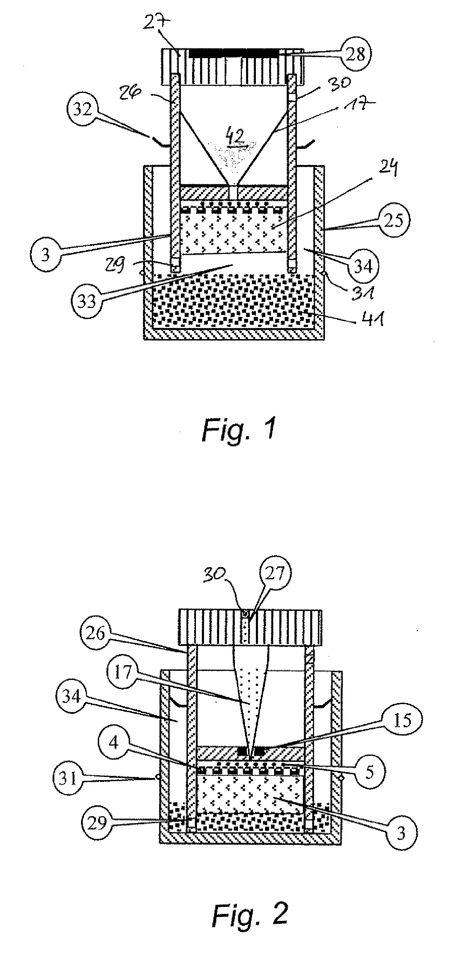

Die in den Fig. 1 und Fig. 2 dargestellte Separationsvorrichtung besteht im Wesentlichen aus einem Probenbehälter 25 (beispielsweise in Form einer zylindrischen Probenküvette), in den - nach der Befüllung mit Vollblut 41 bis zur Markierung 31 - ein Filterkolben 26 eingesetzt wird, der probenseitig ein Filterelement 24 beinhaltet. In einer vom Griffelement 27 verschlossenen Aufnahme des Filterkolbens 26 befindet sich ein spitz zulaufender Filtratsammelbehälter 17 für das abgetrennte Plasma 42, welcher mit der Ausgangsseite des Filterelements 24 in Verbindung steht. Zwischen der Innenwand des Probenbehälters 25 und der Außenwand des Filterkolbens 26 ist eine nach außen durch eine Dichtlippe 32 abgeschlossene Ringkammer 34 ausgebildet, in der sich beim Einführen des Filterkolbens 26 ein auf die Probenflüssigkeit wirkendes Luftpolster ausbildet. Die Dichtlippe 32 kann beispielsweise an der Außenwand des Filterkolbens 26 angeformt oder auch in die Außenwand des Filterkolbens 26 in Form einer Dichtschulter integriert sein.

Optional können zusätzliche Fixierungselemente, beispielsweise Schnappverbindungen oder ähnliches, zwischen Probenbehälter 25 und Filterkolben 26 angebracht sein, welche eine Fixierung des Filterkolbens 26 in der heruntergedrückten Position ermöglichen und hierdurch vermeiden, dass dieser durch den aufgebauten Druck wieder nach oben gedrückt wird und somit das aufgebaute Druckpolster schwächer wird. Besteht durch die Reibung zwischen der Dichtlippe 32 und der Wand des Probenbehälters 25 jedoch eine ausreichend große Reibung, kann bereits diese bewirken, dass der Filterkolben 26 auch ohne zusätzliche Fixierungselemente in der heruntergedrückten Position verbleibt.

Der probenseitige Rand des Filterkolbens 26 ragt über das Filterelement 24 hinaus und bildet eine frontseitige Benetzungskammer 33, in welcher Randausnehmungen oder Zuflussöffnungen 29 angeordnet sind, die eine Strömungsverbindung aus der Ringkammer 34 in die frontseitige Benetzungskammer 33 herstellen. Die Entlüftung des Filterkolbens 26 erfolgt über eine Öffnung 30 im Bereich des Griffelementes 27.

Alternativ können im Boden des Probenbehälters 25 Kerben oder nutförmige Vertiefungen angeordnet sein, die eine Strömungsverbindung von der Ringkammer 34 zur Stirnseite des Filterelements 24 bzw. zur Benetzungskammer 33 herstellen.

In der Ausführungsvariante gemäß Fig. 1 weist das Griffelement 27 des Filterkolbens 26 eine durchstechbare Membran 28 zur Filtratentnahme aus dem Filtratsammelbehälter 17 auf.

Gemäß der in Fig. 2 dargestellten Variante ist der Filtratsammelbehälter 17 am Griffelement 27 des Filterkolbens 26 befestigt und das Griffelement 27 mit dem Filtratsammelbehälter 17 durch Abdrehen oder Abschrauben vom Filterkolben entfernbar, wobei der spitz zulaufende Filtratsammelbehälter 17 (Plasma-Tip) direkt am Eingabeelement eines Analysators angedockt werden kann. Ein Belüftungskanal 30 kann im Griffelement 27 angeordnet sein, der bevorzugt mit einer gasdurchlässigen Membran abgedeckt ist.

Das Filterelement 24 des Filterkolbens 26 ist beispielsweise als Schichtfilter ausgebildet, welches aus einem Tiefenfilter 3, einer Stoppmembran 4 und einem Lateralgitter 5 besteht.

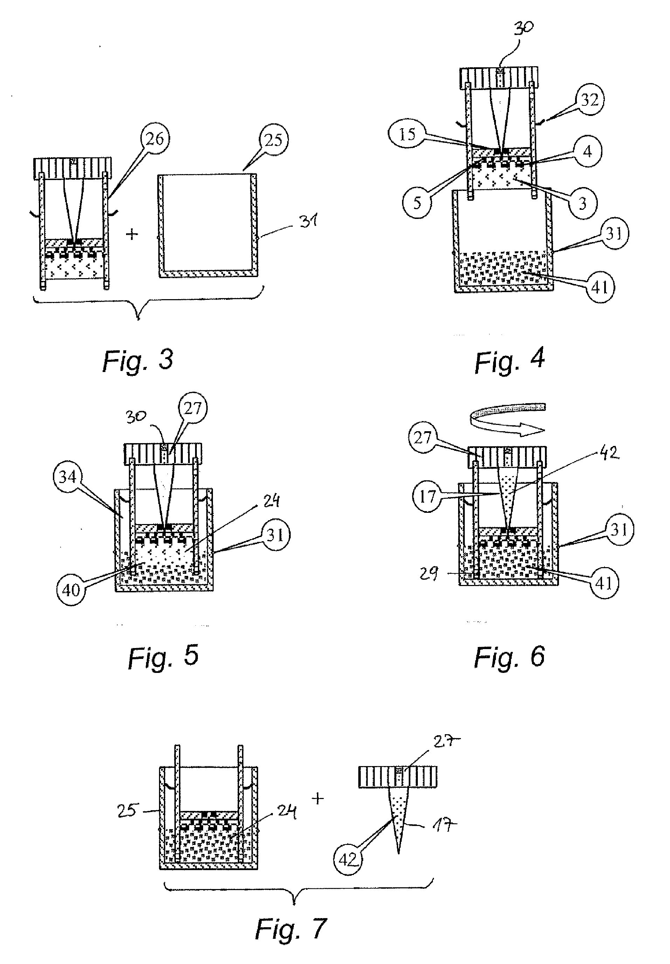

Bei der Plasmagewinnung mit der erfindungsgemäßen Separationsvorrichtung gemäß Fig. 2 kann beispielsweise wie folgt vorgegangen werden:
● Entnahme der Separationsvorrichtung bestehend aus dem Probenbehälter 25 und dem Filterkolben 26 aus einer sterilen Verpackung und Trennen des Filterkolbens 26 vom Probenbehälter 25 (Fig. 3).
● Befüllen des Probenbehälters 25 mit Vollblut 41 bis zur Markierung 31, (beispielsweise 500 µl, vorzugsweise 1 ml), z.B. mit einer Kolbenspritze bzw. einer Pipetiervorrichtung, sowie Ansetzen des Filterkolbens 26 mit den Dichtlippen 32 (Fig. 4)
● Eindrücken des Filterkolbens 26 durch Druckausübung auf das Griffelement 27 bis zum Anschlag am Boden des Probenbehälters 25.
   Hierdurch wird zunächst durch die Abwärtsbewegung des Filterkolbens 26 und durch den hierbei eintretenden luftdichten Abschluss zwischen der an der Außenseite des Filterkolbens 26 befindlichen Dichtlippe 32 und der Innenwand des Probenbehälters 25 ein Luftpolster ausgebildet, welches sich zwischen der Dichtlippe 32 und der Oberfläche der in der Ringkammer 34 befindlichen Blutprobe 41 befindet. Bei weiterem Vorschieben des Filterkolbens 26 wird ein Überdruck im Bereich der Ringkammer 34 ausgebildet.
● Durch das Vorschieben des Filterkolbens 26 wird die Stirnseite des Filterelements 24 in der sich frontseitig ausbildenden Benetzungskammer 33 mit der Blutprobe 41 benetzt und gleichzeitig ein Überdruck in der Ringkammer 34 zwischen der Außenwand des Kolbens 26 und der Innenwand des Probenbehälters 25 erzeugt (Fig. 5).
   Die Zuflussöffnungen 29 am unteren Rand des Filterkolbens 26 erlauben ein Nachfließen der Blutprobe 41. Durch die Entlüftungsöffnung 30 sowie ggf. die luftdurchlässige Deckelmembran 28 wird ein Ansteigen des Plasmapegels im Filtratsammelbehälter 17 ermöglicht.
   Das Tiefenfilter 3 des Filterelements 24 kann z.B. aus bindemittelfreien Glasfasern (typ. FV-2, Fa. Whatman bzw. DE 40 15 589 A1 bzw. EP 0 239 002 A1 Böhringer-Mannheim) mit Rückhaltebereich von 0,5 µm bis 10 µm, besser 1 µm bis 5 µm, vorzugsweise < 3 µm aufgebaut sein. Die roten Blutkörperchen (RBKs) lagern sich dadurch an den feinen Glasfasern des Tiefenfilters 3 an, ohne die Flussrate übermäßig zu beeinflussen bzw. zu zerplatzen.
● Abhängig von Querschnitt des Filterelements 24 im Filterkolben 26 und vom Hämatokrit bildet sich eine "Plasmafront" oder "Plasmafraktion" 40 aus, die ungehindert durch die Stoppmembran 4 treten kann. Dabei werden durch die Stoppmembran 4 die restlichen RBKs herausgefiltert (Fig. 6), welche durch den Tiefenfilter nicht zurückgehalten wurden. Hierzu weist die Stoppmembran 4 in Vergleich zum Tiefenfilter 3 eine deutlich geringere Porengröße auf, beispielsweise Poren mit einem Durchmesser von weniger als 400 nm, bevorzugt mit einem Durchmesser von weniger als 200 nm. Durch die Kombination eines Tiefenfilters 3, welcher aufgrund seiner Porengröße bereits den ganz überwiegenden Teil der Blutzellen zurückhält, aber die Plasmafraktion weitgehend ungestört durchfließen lässt, mit einer nachgeschalteten Stoppmembran 4, welche aufgrund ihrer kleineren Porengröße auch die verbleibenden Blutzellen zuverlässig zurückhält, aber aufgrund ihrer begrenzten Porenzahl ohne den vorgeschalteten Tiefenfilter 4 sehr rasch verstopfen würde, kann eine zuverlässige Abtrennung der Blutzellen ohne rasches Verstopfen des Filters erzielt werden, wodurch die Gewinnung eines ausreichenden Volumens einer Plasmaprobe ermöglicht wird.
   Der mit Hilfe der Filterkenndaten und den geometrischen Verhältnissen der Benetzungskammer 33 und des Ringraumes 34 eingestellte Überdruck von maximal 500 mbar, vorzugsweise 300 mbar, ideal 100 mbar bis 150 mbar, bestimmt die Flussrate und somit die Scherkräfte, die in der Stoppmembran 4 speziell auf die RBKs einwirken. Ein Zerplatzen der RBKs (Hämolyse) kann durch eine Optimierung des Druckvolumens im Ringraum 34 wirksam verhindert werden.
● Das Lateralgitter 5 des Filterelements 24 ermöglicht ein Sammeln und Absaugen des Plasmas 42 nach der Stoppmembran 4 in Richtung Filtratsammelbehälter 17 und verhindert somit ein "Abdichten" durch die Stoppmembran 4. Das Lateralgitter 5 bildet durch seine Gitterstruktur eine nicht durchgängige Auflage für die Stoppmembran 4, so dass Plasma auf der ausgangsseitigen Seite der Stoppmembran 4 in den angeschlossenen Filtratsammelbehälter 17 ausfließen kann. Die Gitterstruktur bewirkt durch die Bildung von Kanälen weiterhin, dass das flächig aus der Stoppmembran 4 hindurchtretende Plasma im Bereich des Filtratsammelbehälters 17 zusammen und in diesen hineinfließen kann.
   *(Diese Funktion des Lateralgitters 5 kann alternativ auch durch Prägen des ausgangsseitigen Bodens des Filterkolbens 26 bzw. ausreichender Rauhigkeit der Oberfläche sichergestellt werden).*
● Die Plasmagewinnung wird i.A. dann beendet, wenn die Plasmafront 40 die Stoppmembran 4 erreicht.
● Bei Hämatokrit < 40% kann die Plasmagewinnung durch vorzeitigen Druckausgleich zum Stillstand kommen.
   *(Alternativ kann die Plasmagewinnung beispielsweise auch dadurch beendet werden, dass der Filtratsammelbehälter 17 eine durch eine hydrophobe Membran geschlossene Entlüftungsöffnung besitzt und bei vollständiger Füllung des Filtratsammelbehälters durch diese verhindert wird, dass weiterhin Filtrat in den Filtratsammelbehälter einfließen kann, wodurch die Plasmagewinnung ebenfalls beendet werden kann).*
● Durch eine optische Kontrolle mittels Markierungen auf dem Filtratsammelbehälter 17 kann überprüft werden, ob die gewünschte Plasmamenge gewonnen werden konnte.
● Entnehmen des Filtratsammelbehälters 17 durch eine Schraubbewegung am Griffstück 27 (Fig. 6, 7).
   *(Alternativ: Die Plasmaentnahme kann auch durch eine perforierte Deckelmembran 28 (**Fig. 1**) erfolgen).*
   *(Alternativ: Die Spitze des Filtratsammelbehälters 17 kann auch als Luer-Kegel ausgestaltet sein).*
● Der Probenbehälter 25 mit dem Rest des Filterkolbens dient als Wastebehälter und kann kontaminationsfrei entsorgt werden (Fig. 7).

## Patentansprüche

1. Separationsvorrichtung zur Abtrennung eines Filtrats von einer Probenflüssigkeit, insbesondere zur Gewinnung von Plasma (42) aus Vollblut (41), mit einem Probenbehälter (25) zur Aufnahme der Probenflüssigkeit und einem in den Probenbehälter (25) dichtend einführbaren Filterkolben (26), der stirnseitig ein Filterelement (24) und an der gegenüberliegenden Seite ein Griffelement (27) aufweist und in seinem Inneren das gewonnene Filtrat aufnimmt, **dadurch gekennzeichnet, dass** nach dem Einsetzen des Filterkolbens (26) in den Probenbehälter (25) zwischen der Innenwand des Probenbehälters (25) und der Außenwand des Filterkolbens (26) eine nach außen durch eine Dichtlippe (32) abgeschlossene Ringkammer (34) ausgebildet ist, in der sich beim Einführen des Filterkolbens (26) in den Probenbehälter (25) ein auf die Probenflüssigkeit wirkendes Luftpolster ausbildet, wobei eine Strömungsverbindung von der Ringkammer (34) zur Stirnseite des Filterelements (24) vorgesehen ist.

2. Separationsvorrichtung nach Anspruch 1, **dadurch gekennzeichnet, dass** der probenseitige Rand des Filterkolbens (26) über das Filterelement (24) ragt und eine frontseitige Benetzungskammer (33) bildet, in welcher Randausnehmungen oder Zuflussöffnungen (29) angeordnet sind, die eine Strömungsverbindung von der Ringkammer (34) in die frontseitige Benetzungskammer (33) herstellen.

3. Separationsvorrichtung nach Anspruch 1 oder 2, **dadurch gekennzeichnet, dass** im Boden des Probenbehälters (25) Kerben oder nutförmige Vertiefungen angeordnet sind, die eine Strömungsverbindung von der Ringkammer (34) zur Stirnseite des Filterelements (24) herstellen.

4. Separationsvorrichtung nach einem der Ansprüche 1 bis 3, **dadurch gekennzeichnet, dass** in einer griffseitigen Aufnahme des Filterkolbens (26) ein in Richtung des Filterelements (24) spitz zulaufender Filtratsammelbehälter (17) angeordnet ist, dessen Spitze das Filterelement (24) kontaktiert.

5. Separationsvorrichtung nach Anspruch 4, **dadurch gekennzeichnet, dass** der Filtratsammelbehälter (17) am Griffelement (27) des Filterkolbens (26) befestigt ist und das Griffelement (27) mit dem Filtratsammelbehälter (17), beispielsweise durch Abdrehen oder Abschrauben, vom Filterkolben (26) entfernbar ist.

6. Separationsvorrichtung nach Anspruch 4, **dadurch gekennzeichnet, dass** das Griffelement (27) des Filterkolbens (26) eine durchstechbare Membran (28) zur Filtratentnahme aus dem Filtratsammelbehälter (17) aufweist.

7. Separationsvorrichtung nach einem der Ansprüche 1 bis 6, **dadurch gekennzeichnet, dass** das Filterelement (24) ein Schichtfilter ist und beispielsweise aus einem Tiefenfilter (3), einer Stoppmembran (4) und einem Lateralgitter (5) besteht.

8. Verfahren zum Abtrennung eines Filtrats von einer Probenflüssigkeit, insbesondere zur Gewinnung von Plasma (42) aus Vollblut (41), **gekennzeichnet durch**
- Bereitstellen eines mit Vollblut (41) gefüllten Probenbehälters (25);
- Einsetzen eines Filterkolbens (26) in den Probenbehälter (25), so dass in einer nach außen **durch** eine Dichtlippe (32) verschlossenen Ringkammer (34) zwischen Probenbehälter (25) und Filterkolben (26) ein unter Überdruck stehendes Luftpolster entsteht, das auf die Probenflüssigkeit wirkt;
- Durchpressen der Probenflüssigkeit **durch** ein im Filterkolben (26) angeordnetes Filterelement (24) mit Hilfe des Überdrucks in der Ringkammer (34), sodass ausgangsseitig des Filterelements (24) Plasma austritt; und
- Sammeln des Plasmas in einem im Filterkolben (26) angeordneten Filtratsammelbehälter (17).

9. Verfahren nach Anspruch 8, **dadurch gekennzeichnet, dass** in der Ringkammer (34) ein Überdruck < 500 mbar, bevorzugt < 300 mbar, besonders bevorzugt zwischen 100 mbar und 150 mbar eingestellt wird.

10. Verfahren nach Anspruch 8 oder 9, **dadurch gekennzeichnet, dass** der Filtratsammelbehälter (17) mit der Plasmaprobe durch eine Schraubbewegung aus dem Filterkolben (26) entnommen wird.
